# EUROPEAN PATENT APPLICATION

(11) **EP 4 834 305 A1**
(43) Date of publication of application: **04.11.2026**
(21) Application number: 25227181.2
(22) Date of filing: 24.12.2025
(51) Int. Cl.: A61K 6/70, C03C 3/097, C03C 10/00, C03C 12/00

(54) **BIOACTIVE GLASS MATERIAL, ORAL CARE COMPOSITION, AND PREPARATION METHODS THEREFOR**

(30) Priority: 31.12.2024 CN 202411979987
(71) Applicant: Jin San Jiang (Zhaoqing) Silicon Material Co. Ltd, Zhaoqing Guangdong 526238 (CN)
(72) Inventor: Huang, Dan, Zhaoqing, Guangdong, 526238 (CN); Wang, Xianwei, Zhaoqing, Guangdong, 526238 (CN); Chen, Guanglong, Zhaoqing, Guangdong, 526238 (CN); Mi, Shuang, Zhaoqing, Guangdong, 526238 (CN); Fan, Ziyi, Zhaoqing, Guangdong, 526238 (CN); Ren, Zhenxue, Zhaoqing, Guangdong, 526238 (CN)
(74) Representative: Petraz, Gilberto Luigi

(57) **Abstract**

The present invention relates to a bioactive glass material, an oral care composition, toothpaste, and preparation methods therefor. The bioactive glass material belongs to a Na₂O-CaO-SiO₂-P₂O₅ system, and includes, in percentage by weight: 40-60% of SiO₂, 1-10% of P₂O₅, 18-30% of CaO, and 10-30% of Na₂O; and an X-ray diffraction pattern of the bioactive glass material includes diffraction peaks at the following 2θ angle values: 20.2±0.2°, 23.8±0.2°, 26.5±0.2°, 26.8±0.2°, 33.6±0.2°, 34.2±0.2°, and 48.6±0.2°. The bioactive glass material provided by the present invention has a special crystal form structure, can dissolve out elements such as calcium, phosphorus, and silicon more quickly to generate a silica glass network and hydroxyapatite, and can react with a tooth surface more quickly to achieve remineralization of a dentin surface, thereby playing the role of alleviating or treating dentin hypersensitivity.

## Description

### TECHNICAL FIELD

The present invention belongs to the technical field of oral care, and more particularly relates to a bioactive glass material, an oral care composition, and preparation methods therefor.

### BACKGROUND

Dentin is an important component of teeth, and dentinal tubules are arranged and distributed in the dentin to radiate from a dental pulp end to an enamel-dentin interface. It is generally believed that when openings of the dentinal tubules on a dentin surface are exposed to cold, heat, sour, sweet, mechanical, or chemical stimuli, nerve endings of the dental pulp are excited, resulting in transient pain, that is, a symptom of dentin hypersensitivity. Studies have shown that compared with individuals without dentin hypersensitivity, individuals with dentin hypersensitivity have a greater number of open dentinal tubules with a larger average diameter. Therefore, the dentin hypersensitivity can be alleviated or treated by occluding the dentinal tubules to reduce the permeability of the dentin.

According to action mechanisms of occlusion, the occlusion can be divided into physical occlusion and chemical occlusion. Chemical occlusion materials can undergo a chemical bonding action with the teeth to play an occlusion role. Currently, materials used for chemical occlusion include bioactive glass, fluorides, and the like.

Among the bioactive glass, a relatively common one is 45S5 bioglass, which is a Na₂O-CaO-SiO₂-P₂O₅ system formed by Na₂O, CaO, SiO₂, and P₂O₅. By adding P₂O₅ to a common Na₂O-CaO-SiO₂ glass system, the material is similar to a natural human bone in elemental composition. Due to the addition of P₂O₅, the bioactivity of the material is increased.

When this bioactive glass is exposed to water or body fluid, the bioglass, with silica as a main network generator, can form a silica gel film during hydrolysis. In addition to adsorbing calcium and phosphorus ions, the gel film can also prevent the exsolution of the calcium and phosphorus ions due to its narrow ion channel, thereby accelerating the repair of the dentinal tubules. The calcium and phosphorus elements in the bioactive glass crystallize on a tooth surface to form mixed hydroxy-carbonate apatite (commonly known as "hydroxyapatite"). The hydroxyapatite deposits on the tooth surface to achieve a mineralization effect to occlude the exposed dentinal tubules.

It can be seen that the silicon, calcium, and phosphorus elements in the bioactive glass have an important effect of occluding the exposed dentinal tubules. The dissolution rates of the silicon, calcium, and phosphorus elements in the bioactive glass determine whether the bioactive glass can react more quickly under the action of the water or body fluid to occlude the exposed dentinal tubules more quickly, thus alleviating or treating the dentin hypersensitivity.

Due to impacts of various factors such as molecular arrangement orders and intermolecular forces, solid substances have different spatial arrangements of molecular lattices, thus forming crystal structures, referred to as crystal forms. Generally, compared with amorphous forms, the crystal forms of the solid substances have lower dissolution rates. The reason is that the solid substances with the crystal forms need to overcome lattice energy during dissolution, which limits the solubility and dissolution rates of the substances. Amorphous structures, due to the lack of long-range ordered structures, do not need to overcome the lattice energy. Therefore, under normal circumstances, the amorphous structures have higher apparent solubility and higher dissolution rates.

### SUMMARY

In view of the above technical problems in the prior art, an objective of the present invention is to provide a bioactive glass material. The bioactive glass material has a special crystal form structure, and compared with existing amorphous bioactive glass during specific applications, can dissolve out elements such as calcium, phosphorus, and silicon more quickly to generate a silica glass network and hydroxyapatite, and can react with a tooth surface more quickly to achieve remineralization of a dentin surface, thereby playing the role of alleviating or treating dentin hypersensitivity.

A second objective of the present invention is to provide preparation methods for the bioactive glass material.

A third objective of the present invention is to provide use of the bioactive glass material in occlusion of a dentinal tubule or in preparation of an oral care composition.

A fourth objective of the present invention is to provide an oral care composition.

A fifth objective of the present invention is to provide toothpaste.

The above objectives of the present invention are achieved through the following technical solutions.

The present invention claims to protect a bioactive glass material, wherein the bioactive glass material belongs to a Na₂O-CaO-SiO₂-P₂O₅ system, and includes, in percentage by weight: 40-60% of SiO₂, 1-10% of P₂O₅, 18-30% of CaO, and 10-30% of Na₂O; and an X-ray diffraction pattern of the bioactive glass material includes diffraction peaks at the following 20 angle values: 33.6±0.2° and 34.2±0.2°.

In some embodiments, the X-ray diffraction pattern of the bioactive glass material includes diffraction peaks at the following 20 angle values: 20.2±0.2°, 23.8±0.2°, 26.5±0.2°, 26.8±0.2°, 33.6±0.2°, 34.2±0.2°, and 48.6±0.2°.

In some embodiments, the X-ray diffraction pattern of the bioactive glass material includes diffraction peaks at the following 2θ angle values: 19.1±0.2°, 20.2±0.2°, 20.7±0.2°, 22.0±0.2°, 23.6±0.2°, 23.8±0.2°, 26.5±0.2°, 26.8±0.2°, 26.9±0.2°, 31.9±0.2°, 32.1±0.2°, 33.6±0.2°, 34.2±0.2°, and 48.6±0.2°.

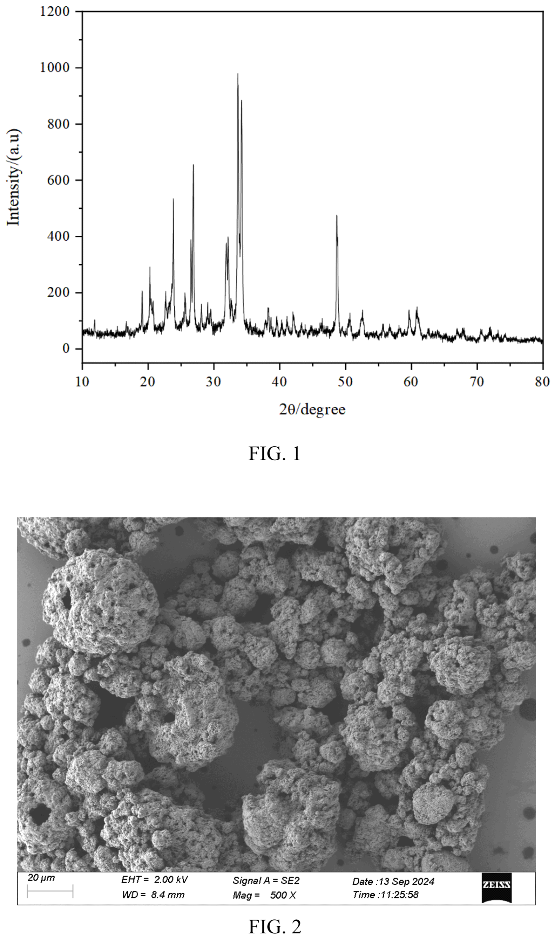

In some embodiments, the bioactive glass material has an X-ray diffraction pattern consistent with FIG. 1, FIG. 4, or FIG. 5.

Preferably, the bioactive glass material includes at least one of the following:
(a) an apparent density of the bioactive glass material being 0.5-1.2 g/mL;
(b) the bioactive glass material including 0.3-2.0% of moisture;
(c) a D50 particle size of the bioactive glass material being 3.5-4.0 µm;
(d) a Brunauer-Emmett-Teller (BET) specific surface area of the bioactive glass material being 10-20 m²/g;
(e) a pore volume of the bioactive glass material being 0.01-0.2 cm³/g;
(f) a pore size of the bioactive glass material being 35-40 nm;
(g) an oil absorption value of the bioactive glass material being 20-100 g/100 g;
(h) a copper consumption value of the bioactive glass material being 2-4 mg; and
(i) a Ganz whiteness of the bioactive glass material being > 98.

Further, the bioactive glass material is prepared and obtained by a first preparation method, a second preparation method, or a third preparation method.

The first preparation method includes the following steps:
(1) subjecting an alkali metal silicate and an inorganic acid to simultaneous acid-base titration, and during the simultaneous titration, maintaining a solution pH at 2-6 and a reaction temperature at 40-95 °C; or
   subjecting an alkali metal silicate and an inorganic acid to simultaneous acid-base titration in a metal salt base solution, and during the simultaneous titration, maintaining a solution pH at 2-6 and a reaction temperature at 40-95 °C; and
(2) after completion of the reaction in step (1), adding calcium oxide, and stirring; adding phosphoric acid for a reaction, and stirring; and then adding a sodium-containing alkali solution, and stirring to prepare and obtain the bioactive glass material.

The second preparation method includes the following steps:
(1) subjecting an alkali metal silicate and phosphoric acid to simultaneous acid-base titration, and during the simultaneous titration, maintaining a solution pH at 3-10 and a reaction temperature at 40-95 °C; or
   subjecting an alkali metal silicate and phosphoric acid to simultaneous acid-base titration in a metal salt base solution, and during the simultaneous titration, maintaining a solution pH at 3-10 and a reaction temperature at 40-95 °C; and
(2) after completion of the reaction in step (1), adding calcium oxide, and stirring; and then adding a sodium-containing alkali solution, and stirring to prepare and obtain the bioactive glass material.

The third preparation method includes the following steps:
S1. at 40-95 °C, mixing a calcium hydroxide slurry and phosphoric acid for a reaction, and maintaining a pH at 7-9 during the reaction; and after completion of the reaction, performing aging treatment to obtain a reaction material solution A;
S2. mixing a silica slurry and calcium oxide, and stirring; and then adding a sodium-containing alkali solution, and stirring to obtain a reaction material solution B; and
S3. mixing the reaction material solution A and the reaction material solution B, and drying to obtain the bioactive glass material.

Further, the present invention claims to protect use of the bioactive glass material in occlusion of a dentinal tubule or in preparation of an oral care composition.

Further, the present invention claims to protect an oral care composition, which includes, in percentage by weight: 0.1-20% of the bioactive glass material and an orally acceptable auxiliary material.

Further, the present invention claims to protect toothpaste, which includes, in parts by weight: 1-10 parts of the above-mentioned bioactive glass material, 20.5-26.5 parts of a thickener, 45-60 parts of a humectant, 11-20 parts of an abrasive, 1-5 parts of a foaming agent, 0-3 parts of a colorant, 0-3 parts of a flavoring agent, 0-0.5 part of a sweetener, and 0-0.5 part of an antimicrobial agent.

Further, the present invention claims to protect toothpaste, which includes, in parts by weight: 1-10 parts of the above-mentioned bioactive glass material, 20.5-26.5 parts of a thickener, 45-60 parts of a humectant, 11-20 parts of an abrasive, 1-5 parts of a foaming agent, 0.1-3 parts of a colorant, 0.1-3 parts of a flavoring agent, 0.1-0.5 part of a sweetener, and 0.1-0.5 part of an antimicrobial agent.

Further, the present invention claims to protect toothpaste, which includes, in percentage by weight: 0.95% of carbomer 940, 21.25% of PEG-400, 0.2% of sodium saccharin, 0.2% of methylparaben, 55% of glycerol, 3% of ZI-165, 11% of core-shell silica, 2% of K12, 5% of the bioactive glass material, 0.5% of titanium dioxide, and 0.9% of an essence.

Compared with the prior art, the present invention has the following beneficial effects.
(1) The present invention provides a bioactive glass material with a specific crystal form structure, which, during practical applications, can dissolve out elements such as calcium, phosphorus, and silicon more quickly to generate a silica glass network and hydroxyapatite, and can react with a tooth surface more quickly to achieve remineralization of a dentin surface, thereby playing the role of alleviating or treating dentin hypersensitivity.
(2) The bioactive glass material provided by the present invention has a pH of 8.0-10.0, and compared with bioglass with a pH of greater than 10, has a wider range of application, fewer restrictions of toothpaste formulations, and lower irritation to oral mucosa. The bioactive glass material provided by the present invention has the advantages of good biocompatibility, no toxicity, and a low cost.

### BRIEF DESCRIPTION OF DRAWINGS

FIG. 1 shows an X-ray diffraction pattern of a bioactive glass material prepared in Example 1.
FIG. 2 and FIG. 3 show scanning electron microscopy (SEM) images of the bioactive glass material prepared in Example 1.
FIG. 4 shows an X-ray diffraction pattern of a bioactive glass material prepared in Example 5.
FIG. 5 shows an X-ray diffraction pattern of a bioactive glass material prepared in Example 6.
FIG. 6 shows an X-ray diffraction pattern of a bioactive glass material of Comparative Example 1.
FIG. 7 shows an X-ray diffraction pattern of a bioactive glass material of Comparative Example 2.
FIG. 8, FIG. 9, and FIG. 10 respectively show X-ray diffraction patterns of products after artificial saliva mineralization of the bioactive glass materials of Example 1, Comparative Example 1, and Comparative Example 2.
FIG. 11, FIG. 12, and FIG. 13 respectively show experimental images of a dentinal tubule mineralization and occlusion experiment using bioglass toothpaste prepared from the bioactive glass materials of Example 1, Comparative Example 1, and Comparative Example 2.

### DETAILED DESCRIPTION OF EMBODIMENTS

In the following description, certain specific details are set forth to provide a thorough understanding of various embodiments of the present invention. However, those skilled in the art will understand that the present invention may be implemented without using these details. The description of various embodiments below is made based on an understanding of the following contents that the present disclosure is to be regarded as an example of subject contents requiring protection, and is not intended to limit the appended claims to the specific embodiments described. Headings used throughout the present disclosure are provided solely for convenience and are not to be construed as limiting the claims in any way. The embodiments described under any headings may be combined with embodiments described under any other headings.

### [Composition and crystal form]

Due to impacts of various factors such as molecular arrangement orders, molecular structural configurations, conformations, intermolecular forces, and eutectic substances, solid substances have different spatial arrangements of molecular lattices, thus forming two or more distinct crystal structures. Such phenomenon is referred to as a "polymorphism phenomenon" or an "allomorphism phenomenon". The "polymorphism phenomenon" is widely found in solids. Different crystal forms of the same substance may have differences in physicochemical properties, and for example, may be significantly different in terms of reaction rate, stability, solubility, dissolution rate, and bioavailability.

The crystal forms can be determined by various technical means, such as X-ray diffraction (XRD), X-ray powder diffraction (XRPD), infrared absorption spectroscopy (IR), melting point method, differential scanning calorimetry (DSC), thermogravimetric analysis (TGA), nuclear magnetic resonance, Raman spectroscopy, dissolution calorimetry, scanning electron microscopy (SEM), quantitative analysis, solubility, and dissolution rate, etc.

The X-ray diffraction (XRD) can detect information such as changes in crystal form, degree of crystallinity, and crystal structure state, which is a commonly used means for identifying the crystal forms. Crystalline substances and non-crystalline (amorphous) substances can be distinguished. XRD patterns of different crystal forms show differences in the number, position, intensity, and shape of diffraction peaks, and thus can be used as fingerprint patterns of crystals to identify and distinguish different crystal forms. More specifically, differences in crystal form structure are primarily determined by the positions of the diffraction peaks in the XRD patterns. Therefore, in some embodiments, the distinction between bioactive glass provided by the present invention and existing bioactive glass is characterized by having certain characteristic peak positions characterized by 2θ angle values and XRD patterns, which are substantially as shown in XRD patterns provided in the drawings of the present invention. Based on a conventional understanding of those of ordinary skill in the art, the 20 angle values in the XRD patterns may have an experimental error, and the 20 angle values in the XRD patterns may be slightly different between different instruments and different samples. Therefore, the 20 angle values provided by the present invention should not be regarded as absolute. According to instrument conditions used in tests of the present invention, the characteristic diffraction peaks characterized by the 20 angle values have an error tolerance of ±0.2°.

Furthermore, when referring to, for example, an XRD pattern or an XRPD pattern, "consistent with FIG..." does not mean completely identical to the drawings provided or described herein. When considered by those of ordinary skill in the art, patterns falling within the experimental error or the above-mentioned error tolerance should also be taken into account.

A bioactive glass material provided by the present invention has a specific crystal form. Through research, the inventor has surprisingly found that compared with existing amorphous bioactive glass, when used for mineralizing and occluding dentinal tubules under the action of water or body fluid, the bioactive glass material with a specific crystal form provided by the present invention can dissolve out elements such as calcium, phosphorus, and silicon in the bioactive glass material more quickly to generate a silica glass network and hydroxyapatite, and thus can react with a tooth surface more quickly to achieve remineralization of a dentin surface, thereby playing the role of alleviating or treating dentin hypersensitivity.

The bioactive glass material provided by the present invention can be distinguished by those of ordinary skill in the art according to diffraction peaks at the following 20 angle values: 33.6±0.2° and 34.2±0.2°. More specifically, in some more detailed X-ray diffraction pattern data, an X-ray diffraction pattern of the bioactive glass material provided by the present invention includes diffraction peaks at the following 2θ angle values: 20.2±0.2°, 23.8±0.2°, 26.5±0.2°, 26.8±0.2°, 33.6±0.2°, 34.2±0.2°, and 48.6±0.2°. More specifically, diffraction peaks at the following 20 angle values are included: 19.1±0.2°, 20.2±0.2°, 20.7±0.2°, 22.0±0.2°, 23.6±0.2°, 23.8±0.2°, 26.5±0.2°, 26.8±0.2°, 26.9±0.2°, 31.9±0.2°, 32.1±0.2°, 33.6±0.2°, 34.2±0.2°, and 48.6±0.2°. More specifically, the bioactive glass material provided by the present invention has an X-ray diffraction pattern substantially consistent with FIG. 1, FIG. 4, or FIG. 5.

Further, in some embodiments, the bioactive glass material provided by the present invention includes, in percentage by weight: 40-60% of SiO₂, 1-10% of P₂O₅, 18-30% of CaO, and 10-30% of Na₂O.

As non-limiting examples of the content of SiO₂ in the bioactive glass material, the weight percentage of SiO₂ may be 42%, 44%, 46%, 48%, 50%, 52%, 54%, 56%, 58%, etc., or within an interval range formed by any of the above numerical values, for example 42-50%, 52-58%, etc., which is not limited in the present invention.

As non-limiting examples of the content of P₂O₅ in the bioactive glass material, the weight percentage of P₂O₅ may be 1%, 2%, 3%, 4%, 5%, 6%, 7%, 8%, 9%, etc., or within an interval range formed by any of the above numerical values, for example, 1-4%, 3-10%, etc., which is not limited in the present invention.

As non-limiting examples of the content of CaO in the bioactive glass material, the weight percentage of CaO may be 20%, 21%, 22%, 23%, 24%, 25%, 26%, 27%, 28%, 29%, etc., or within an interval range formed by any of the above numerical values, for example 20-25%, 23-29%, etc., which is not limited in the present invention.

As non-limiting examples of the content of Na₂O in the bioactive glass material, the weight percentage of Na₂O may be 12%, 14%, 16%, 18%, 20%, 22%, 24%, 26%, 28%, etc., or within an interval range formed by any of the above numerical values, for example, 12-20%, 16-28%, etc., which is not limited in the present invention.

Illustrative non-limiting examples conforming to the bioactive glass material of the present invention may have the following features: in one or more embodiments, the bioactive glass material may have an apparent density of 0.5-1.2 g/mL; and more specifically, the apparent density may be 0.5-0.7 g/mL. In one or more embodiments, the bioactive glass material may include 0.3-2.0% of moisture; and more specifically, includes 0.3-1.0% of moisture. In one or more embodiments, a D50 particle size of the bioactive glass material may be 3.5-4.0 µm; and more specifically, the D50 particle size may be 3.5-3.7 µm. In one or more embodiments, a BET specific surface area of the bioactive glass material may be 10-20 m²/g. In one or more embodiments, a pore volume of the bioactive glass material may be 0.01-0.2 cm³/g; and more specifically, the pore volume may be 0.1-0.2 cm³/g. In one or more embodiments, a pore size of the bioactive glass material may be 10-40 nm; and more specifically, the pore size may be 35-40 nm. In one or more embodiments, an oil absorption value of the bioactive glass material may be 20-100 g/100 g; and more specifically, the oil absorption value may be 70-85 g/100 g. In one or more embodiments, a copper consumption value of the bioactive glass material is 2-4 mg. In one or more embodiments, a Ganz whiteness of the bioactive glass material is > 98; and more specifically, the Ganz whiteness may be 98.5-99.9.

In one or more embodiments, a pH of the bioactive glass material may be 8.5-10. Compared with existing bioactive glass materials with a pH generally exceeding 10, controlling the pH of the bioactive glass material within 8.5-10 has the advantage of a wider range of application, and has fewer formulation restrictions when prepared into an oral composition. Furthermore, controlling the pH within 8.5-10 also has the advantage of lower irritation to oral mucosa.

### [Preparation methods]

The bioactive glass material with a specific crystal form structure provided by the present invention can be prepared and obtained by various methods.

According to non-limiting examples conforming to the preparation methods of the present invention, the bioactive glass material may be obtained by the following first preparation method:
(1) subjecting an alkali metal silicate and an inorganic acid to simultaneous acid-base titration, and during the simultaneous titration, maintaining a solution pH at 2-6 and a reaction temperature at 40-95 °C; or
   subjecting an alkali metal silicate and an inorganic acid to simultaneous acid-base titration in a metal salt base solution, and during the simultaneous titration, maintaining a solution pH at 2-6 and a reaction temperature at 40-95 °C; and
(2) after completion of the reaction in step (1), adding calcium oxide, and stirring; adding phosphoric acid for a reaction, and stirring; and then adding a sodium-containing alkali solution, and stirring to prepare and obtain the bioactive glass material.

In step (1), maintaining the solution pH and the reaction temperature within specific ranges promotes the formation of porous silica gel, which can then react more thoroughly with the calcium oxide introduced in the second step. Deviating beyond these ranges leads to incomplete reaction between the silica gel and calcium oxide, compromising the uniformity and stability of the bioglass network. In step (2), the addition of calcium oxide, phosphoric acid, and a sodium-containing alkaline solution further generates calcium hydroxyphosphate, calcium silicate, and sodium silicate, ultimately yielding a bioactive glass material with uniformly distributed elements.

In the above-mentioned method, as a non-limiting example of the calcium oxide, commercially available calcium oxide commonly used in the art may be used as a substitute. More specifically, a calcium salt (such as calcium carbonate, etc.) may also be used and sintered to obtain sintered calcium oxide. More specifically, in some embodiments, a heat source (such as a muffle furnace, etc.) of not lower than 800 °C, not lower than 850 °C, not lower than 900 °C, not lower than 950 °C, not lower than 1,000 °C, not lower than 1,100 °C, or not lower than 1,200 °C may be used for continuous calcination of the calcium salt. More specifically, a sintering temperature is 900-1,200 °C. More specifically, the sintering temperature is 1,000-1,100 °C. A conversion rate of the sintered calcium oxide using the above-mentioned method may be ≥ 96%; more preferably, the conversion rate is ≥ 97%; more preferably, the conversion rate is ≥ 98%; and most preferably, the conversion rate is 100%. At this conversion rate, the sintered calcium oxide has higher activity. A calculation method for the conversion rate is that conversion rate Y = △m/44%m, wherein △m refers to a mass difference of the calcium carbonate before and after the calcination; and m refers to a mass of the calcium carbonate. More specifically, the calcium salt may be calcium carbonate, calcium chloride, calcium bicarbonate, or a mixture thereof.

As non-limiting examples of the alkali metal silicate, the alkali metal silicate may be selected from sodium silicate, potassium silicate, or a mixture thereof. In some non-limiting examples, a modulus of the alkali metal silicate may be 1-3.8, and a concentration may be 1-2.9 N. More specifically, the modulus of the alkali metal silicate may be 3-3.6, and the concentration may be 1.2-2.6 N. More specifically, the modulus of the alkali metal silicate may be 3.1-3.5, and the concentration may be 1.2-2.2 N. The use of the alkali metal silicate, within conditions of preferred modulus and concentration, facilitates the formation of porous silica gel, which in turn enables the production of a bioactive glass material with a more uniform elemental distribution and, ultimately, markedly enhanced dissolution rate and occlusion effect. As non-limiting examples of the inorganic acid, the inorganic acid may be sulfuric acid, hydrochloric acid, nitric acid, phosphoric acid, or a mixture thereof. In some non-limiting examples, a concentration of the inorganic acid may be 2-12 N. More specifically, the concentration of the inorganic acid may be 4-10 N. Within this preferred concentration of the inorganic acid, the dissolution rate and occlusion effect of the bioactive glass are further enhanced.

As a non-limiting example, the metal salt base solution may be a metal salt base solution formed by the combination of an anion of the above inorganic acid and an alkali metal of the alkali metal silicate, including but not limited to sodium sulfate, etc. In some non-limiting examples, the sodium-containing alkali solution is mainly used for providing a sodium element in bioactive glass, and may be, for example, sodium hydroxide, etc.

In the step (1) of the above-mentioned method, as non-limiting examples of a reaction time, the reaction time of the simultaneous acid-base titration is not lower than 0.5 h, not lower than 1.0 h, or not lower than 1.5 h. In some embodiments, the reaction time of the simultaneous acid-base titration is 0.5-1.5 h.

In the step (1) of the above-mentioned method, as non-limiting examples of the reaction temperature, the reaction temperature may be 45 °C, 50 °C, 55 °C, 60 °C, 65 °C, 70 °C, 75 °C, 80 °C, 85 °C, 90 °C, etc. More specifically, the reaction temperature may be 45-90 °C. More specifically, the reaction temperature may be 60-90 °C.

In the step (2) of the above-mentioned method, after the calcium oxide is added, a stirring time is not lower than 10 min, not lower than 15 min, not lower than 20 min, not lower than 30 min, etc.; and more specifically, the stirring time is 10-30 min. A rotation speed of the stirring may be 100 rpm (revolutions per minute), 300 rpm, 500 rpm, 700 rpm, 900 rpm, 1,000 rpm, etc. More specifically, the rotation speed of the stirring is 100-1,000 rpm. More specifically, the rotation speed of the stirring is 400-800 rpm.

In the step (2) of the above-mentioned method, a concentration of the added phosphoric acid may be 30-40%, and a reaction time is not lower than 0.5 h, not lower than 1.0 h, or not lower than 1.5 h. More specifically, the reaction time is 0.5-1.5 h. More specifically, the reaction time is 0.5-1 h. After the addition of phosphoric acid and completion of the reaction, stirring is performed continuously, and a stirring time is not lower than 10 min, not lower than 15 min, not lower than 20 min, not lower than 30 min, etc.; and more specifically, the stirring time is 10-40 min. More specifically, the stirring time is 20-40 min. A rotation speed of the stirring may be 100 rpm, 300 rpm, 500 rpm, 700 rpm, 900 rpm, 1,000 rpm, etc. More specifically, the rotation speed of the stirring is 100-1,000 rpm. More specifically, the rotation speed of the stirring is 400-800 rpm.

In the step (2) of the above-mentioned method, after the sodium-containing alkali solution is added, a stirring time is not lower than 10 min, not lower than 15 min, not lower than 20 min, not lower than 30 min, etc.; and more specifically, the stirring time is 10-40 min. More specifically, the stirring time is 20-40 min. A rotation speed of the stirring may be 100 rpm, 300 rpm, 500 rpm, 700 rpm, 900 rpm, 1,000 rpm, etc. More specifically, the rotation speed of the stirring is 100-1,000 rpm. More specifically, the rotation speed of the stirring is 400-800 rpm.

Further, the present invention provides a second method for preparing the bioactive glass material, which includes the following steps:
(1) subjecting an alkali metal silicate and phosphoric acid to simultaneous acid-base titration, and during the simultaneous titration, maintaining a solution pH at 3-10 and a reaction temperature at 40-95 °C; or
   subjecting an alkali metal silicate and phosphoric acid to simultaneous acid-base titration in a metal salt base solution, and during the simultaneous titration, maintaining a solution pH at 3-10 and a reaction temperature at 40-95 °C; and
(2) after completion of the reaction in step (1), adding calcium oxide, and stirring; and then adding a sodium-containing alkali solution, and stirring to prepare and obtain the bioactive glass material.

In the step (1) of the above-mentioned method, as non-limiting examples of the pH, the pH may be 4, 5, 6, 7, 8, 9, etc., or within an interval range formed by any of the above numerical values, for example, 4-7, 8-10, etc., which is not limited in the present invention.

In the step (1) of the above-mentioned method, as non-limiting examples of the reaction temperature, the reaction temperature may be 45 °C, 50 °C, 55 °C, 60 °C, 65 °C, 70 °C, 75 °C, 80 °C, 85 °C, 90 °C, etc. More specifically, the reaction temperature may be 45-90 °C. More specifically, the reaction temperature may be 60-90 °C.

In the step (1) of the above-mentioned method, as non-limiting examples of the alkali metal silicate, a modulus of the alkali metal silicate may be 1-3.8, and a concentration may be 1-2.9 N. More specifically, the modulus of the alkali metal silicate may be 2-3.6, and the concentration may be 2-2.6 N. More specifically, the modulus of the alkali metal silicate may be 3-3.5, and the concentration may be 2.1-2.4 N. As non-limiting examples of the inorganic acid, a concentration of the inorganic acid may be 2-12 N. More specifically, the concentration of the inorganic acid may be 4-8 N. More specifically, the concentration of the inorganic acid may be 5-7 N.

Other specific process parameters of the second method for preparing the bioactive glass material provided by the present invention may refer to the above-mentioned first method for preparing the bioactive glass material.

Further, the present invention provides a third method for preparing the bioactive glass material, which includes the following steps:
S1. at 40-95 °C, mixing a calcium hydroxide slurry and phosphoric acid for a reaction, and maintaining a pH at 7-9 during the reaction; and after completion of the reaction, performing aging treatment to obtain a reaction material solution A;
S2. mixing a silica slurry and calcium oxide, and stirring; and then adding a sodium-containing alkali solution, and stirring to obtain a reaction material solution B; and
S3. mixing the reaction material solution A and the reaction material solution B, drying, and crushing to obtain the bioactive glass material.

As non-limiting examples of the reaction temperature in the step S1, the reaction temperature may be 60-90 °C; and more specifically, may be 80-90 °C. As non-limiting examples of the calcium hydroxide slurry, a concentration of the calcium hydroxide slurry may be 0.5-1 mol/L; and more specifically, may be 0.6-0.8 mol/L. As non-limiting examples of the phosphoric acid, a concentration of the phosphoric acid may be 30-40%; and more specifically, the concentration of the phosphoric acid may be 35-40%.

In the step S2, the sodium-containing alkali solution is mainly used for providing a sodium element in bioactive glass, and may be, for example, sodium hydroxide, etc. In the step S2, a stirring time is not lower than 10 min, not lower than 15 min, not lower than 20 min, not lower than 30 min, etc.; and more specifically, the stirring time is 10-40 min. More specifically, the stirring time is 20-40 min. A rotation speed of the stirring may be 100 rpm, 300 rpm, 500 rpm, 700 rpm, 900 rpm, 1,000 rpm, etc. More specifically, the rotation speed of the stirring is 100-1,000 rpm. More specifically, the rotation speed of the stirring is 400-800 rpm. In the step S2, after the sodium-containing alkali solution is added, a stirring time is not lower than 1 h, not lower than 2 h, not lower than 3 h, not lower than 4 h, etc.; and more specifically, the stirring time is 1-4 h.

### [Oral care composition]

The bioactive glass material provided by the present invention may be used in preparation of an oral care composition for occluding a dentinal tubule.

The oral care composition of the present invention may be in any form known in the art that is suitable for oral care. As non-limiting examples of the oral care composition, the oral care composition may be toothpaste, a gel, a mouthwash, dental floss, a dentifrice used for cleaning an oral surface such as a paste, a powder, a tablet, or a liquid preparation, dental gum, a dental patch, an oral spray, a tooth powder, foam, gum, a lip balm, sponge, a mouth rinse, chewing gum, or a denture product, etc.

An orally acceptable auxiliary material refers to an ingredient used in an oral care composition that is suitable for a physiological environment of the oral cavity and does not cause excessive side effects to the oral cavity.

The present invention provides an oral care composition, which includes, in percentage by weight, 0.1-20% of the above-mentioned bioactive glass material and an orally acceptable auxiliary material. The weight percentage of the bioactive glass material in the oral care composition may be 0.2%, 0.5%, 1%, 1.5%, 2%, 2.5%, 3%, 3.5%, 4%, 4.5%, 5%, 8%, 10%, 15%, 18%, etc., or within an interval range formed by any of the above numerical values, for example, 1.5-3%, 2-5%, etc., which is not limited in the present invention.

As non-limiting examples, the auxiliary material may be a conventional ingredient used for oral care composition in the art, including but not limited to one or more of a thickener, a sweetener, an antimicrobial agent, an abrasive, a humectant, a pigment or colorant, a flavoring agent, and a foaming agent.

Exemplary thickeners include, but are not limited to: carbomer (such as carbomer 940), polyethylene glycol (such as PEG-400), hydroxyethyl cellulose, carboxymethyl cellulose and its salts (e.g., sodium carboxymethyl cellulose), carrageenan, carboxyvinyl polymers, xanthan gum, carrageenan, gelatin, branched starch, sodium alginate, etc. In certain embodiments, the thickener includes one or more of xanthan gum, carrageenan, or sodium carboxymethyl cellulose. Preferably, the thickener is selected from carbomer and/or polyethylene glycol.

Exemplary sweeteners include, but are not limited to: sodium saccharin, flavoring oils such as spearmint oil, peppermint oil, wintergreen oil, sassafras oil, clove oil, salvia officinalis oil, eucalyptus oil, cinnamon oil, lemon oil, and orange peel oil, methyl salicylate, and eugenol. Preferably, the sweetener is selected from sodium saccharin.

Exemplary antimicrobial agents include, but are not limited to: methylparaben, zinc oxide, stannous chloride, tetrahydrocurcumin, cetylpyridinium chloride, triclosan, etc. Preferably, the antimicrobial agent is selected from methylparaben.

Exemplary humectants include, but are not limited to: glycerol, sorbitol, xylitol, and propylene glycol, etc. Preferably, the humectant is selected from glycerol.

Various other optional ingredients well known in practice may be added to a toothpaste composition of the present invention to improve general aesthetic appearance. These ingredients include a pigment, a dye, a reagent for forming colored spots, etc.

Exemplary flavoring agents may be those conventionally used in the art, including but not limited to: a wintergreen essence, a peppermint essence, a spearmint essence, a sassafras essence, and a clove essence, etc.

Exemplary foaming agents include, but are not limited to: sodium lauryl sulfate, cocamidopropyl betaine, alkyl glycosides, alkyl sulfonates, alkylbenzene sulfonates, etc.

Exemplary abrasives include, but are not limited to: silica, calcium carbonate, calcium bicarbonate, calcium pyrophosphate, etc.

Further, in some embodiments, the present invention provides toothpaste, which includes, in parts by weigh: 1-10 parts of the bioactive glass material, 20.5-26.5 parts of a thickener, 45-60 parts of a humectant, 11-20 parts of an abrasive, 1-5 parts of a foaming agent, 0-3 parts of a colorant, 0-3 parts of a flavoring agent, 0-0.5 part of a sweetener, and 0-0.5 part of an antimicrobial agent. Further, the toothpaste includes, in parts by weight: 1-10 parts of the bioactive glass material, 20.5-26.5 parts of the thickener, 45-60 parts of the humectant, 11-20 parts of the abrasive, 1-5 parts of the foaming agent, 0.1-3 parts of the colorant, 0.1-3 parts of the flavoring agent, 0.1-0.5 part of the sweetener, and 0.1-0.5 part of the antimicrobial agent.

### Raw material information and test methods

Method for testing the apparent density: QB/T2346-2015.

Method for testing the copper consumption: JSJ-C-ZG-36: (1) 2 pieces of copper sheets were selected, cleaned with distilled water, dried with a blower, placed in a dryer for 15 min, and then taken out with tweezers. The weight W1 (unit: mg) of the two pieces of copper sheets before abrasion was weighed, respectively. After the weighing, the copper sheets were placed in a material tank of a hard particle tester, respectively.

(2) 20 g of a bioactive glass material sample was accurately weighed and evenly dispersed in 120 g of a sorbitol solution, a resulting abrasive slurry was transferred to the material tank of the hard particle tester, the hard particle tester was turned on, and the copper sheets were continuously abraded with an abrasive head for 10,000 times in the test slurry. After the completion of abrasion, the copper sheets were taken out with tweezers, rinsed with distilled water, dried with a blower, placed in a dryer for 15 min, and then taken out with tweezers. The weight W2 (unit: mg) of the copper sheets after the abrasion was weighed, respectively. (3) A copper consumption abrasion value was calculated as W=W1-W2 (unit: mg), the deviation of parallel experiment results was not allowed to exceed 20%, and an average value was taken as a final result.

Method for testing the Ganz whiteness: QB/T2346-2015.

Method for testing the moisture: QB/T2346-2015.

Method for testing the particle size: GBT19087-2016.

Method for testing the BET specific surface area, the pore volume, and the pore size: The specific surface area, pore volume, and pore size of amorphous silica particles were tested using a JW-BK112 static nitrogen adsorption instrument.

Method for testing the oil absorption value: ASTM D281.

Method for testing the pH: QB/T2346-2015.

Bioactive glass 4516, Novamin 4516, 3M.

ZI-165: Silica, Jin San Jiang.

Core-shell type: Core-shell silica, Jin San Jiang.

K12: Sodium lauryl sulfate, Jiangsu Youyang Pharmaceutical Co., Ltd.

### Example 1

(1) Sintering of calcium carbonate: A quantity of analytically pure calcium carbonate was weighed, placed in a muffle furnace for calcination at 1,000°C, and then cooled to room temperature to achieve a conversion rate of 100% to obtain sintered calcium oxide. A calculation formula for the conversion rate is as follows: Y = △m/44%m, wherein △m refers to a mass difference of the calcium carbonate before and after the calcination; and m refers to a mass of the calcium carbonate.
(2) Simultaneous acid-base titration was performed at a reaction temperature of 90°C, wherein a pH was maintained at 2.5 during the simultaneous acid-base titration, and a reaction time was 1 h. A base used was sodium silicate with a modulus of 3.5 and a concentration of 1.2 N, and based on silica generated by the alkali metal silicate accounting for 50% of a total mass of a bioactive glass material, a corresponding mass of the alkali metal silicate was added. An acid used was sulfuric acid with a concentration of 8 N.
(3) After completion of the reaction, the sintered calcium oxide obtained in step (1) was added, with an addition amount of the sintered calcium oxide accounting for 22% of the total mass of the bioactive glass material, and then stirred evenly at a rotation speed of 600 rpm for 10 min; subsequently, diluted phosphoric acid was added for a reaction time of 0.6 h, wherein a concentration of the diluted phosphoric acid was 30%, and based on generated phosphorus pentoxide accounting for 5% of the total mass of the bioactive glass material, a corresponding mass of the diluted phosphoric acid was added; after completion of the reaction, stirring was performed continuously at 600 rpm for 30 min, and subsequently, sodium hydroxide was added, wherein based on generated sodium oxide as sodium introduced by the sodium hydroxide, the sodium oxide accounted for 8.2% of the total mass of the bioactive glass material; and stirring was performed continuously at a rotation speed of 600 rpm for 30 min, followed by discharging.
(4) A material solution discharged in the step (3) was subjected to spray drying at an air inlet temperature of 300°C and an air outlet temperature of 120 °C, and then crushed to a required particle size to obtain the bioactive glass material.

After analysis and identification by X-ray diffraction (XRD) using a Bruker D8A A25 X-ray diffractometer with Cu-Kα radiation, the bioactive glass material prepared in this example has characteristic peaks expressed at the following 2θ angles: 2θ=19.1±0.2°, 2θ=20.2±0.2°, 2θ=23.8±0.2°, 2θ=26.5±0.2°, 2θ=26.9±0.2°, 2θ=31.94±0.2°, 2θ=32.1±0.2°, 2θ=33.6±0.2°, 2θ=34.2±0.2°, 2θ=48.6±0.2°. An X-ray diffraction pattern of the bioactive glass material prepared in this example is shown in FIG. 1. The bioactive glass material prepared in Example 1 belongs to a Na₂O-CaO-SiO₂-P₂O₅ system, and includes, in percentage by weight: 50% of SiO₂, 5% of P₂O₅, 22% of CaO, and 23% of Na₂O.

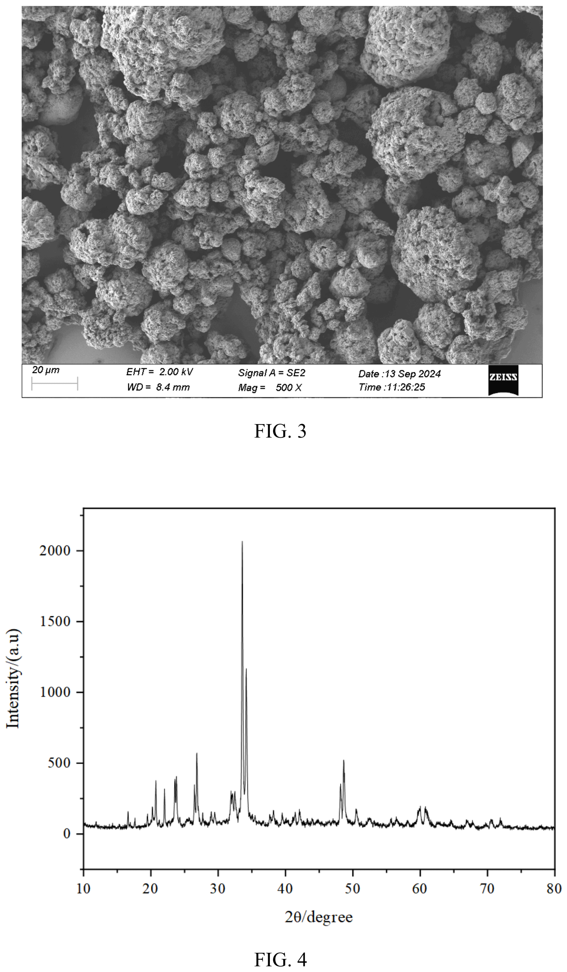

SEM images of the bioactive glass material prepared in Example 1 are shown in FIG. 2 and FIG. 3.

### Example 2

(1) Sintering of calcium carbonate: A quantity of analytically pure calcium carbonate was weighed, placed in a muffle furnace for calcination at 1,000 °C, and then cooled to room temperature to achieve a conversion rate of 100% to obtain sintered calcium oxide. A calculation formula for the conversion rate is as follows: Y = △m/44%m, wherein △m refers to a mass difference of the calcium carbonate before and after the calcination; and m refers to a mass of the calcium carbonate.
(2) A metal salt base solution at a volume proportion of 20% (based on a reactor volume of 5 L) was added into a reaction tank, wherein the metal salt base solution was sodium sulfate, and based on generated sodium oxide as sodium introduced by the sodium sulfate, the sodium oxide accounted for 3% of a total mass of a bioactive glass material; and at a reaction temperature of 80 °C, simultaneous acid-base titration was performed, wherein a pH was maintained at 3.5 during the simultaneous acid-base titration, and a reaction time was 1 h. A base used was sodium silicate with a modulus of 3.3 and a concentration of 1.5 N, and based on silica generated by the alkali metal silicate accounting for 40% of the total mass of the bioactive glass material, a corresponding mass of the alkali metal silicate was added. An acid used was sulfuric acid with a concentration of 4 N.
(3) After completion of the reaction, the sintered calcium oxide obtained in step (1) was added, with an addition amount of the sintered calcium oxide accounting for 27% of the total mass of the bioactive glass material, and then stirred evenly at a rotation speed of 600 rpm for 15 min; subsequently, diluted phosphoric acid was added for a reaction time of 0.5 h, wherein a concentration of the diluted phosphoric acid was 33%, and based on generated phosphorus pentoxide accounting for 5% of the total mass of the bioactive glass material, a corresponding mass of the diluted phosphoric acid was added; after completion of the reaction, stirring was performed continuously at 600 rpm for 30 min, and subsequently, sodium hydroxide was added, wherein based on sodium oxide as sodium introduced by the sodium hydroxide, the sodium oxide accounted for 12.5% of the total mass of the bioactive glass material; and stirring was performed continuously at a rotation speed of 600 rpm for 30 min, followed by discharging.
(4) A material solution discharged in the step (3) was subjected to spray drying at an air inlet temperature of 300 °C and an air outlet temperature of 120 °C, and then crushed to a required particle size to obtain the bioactive glass material.

After analysis and identification by X-ray diffraction (XRD) using a Bruker D8A A25 X-ray diffractometer with Cu-Kα radiation, the bioactive glass material prepared in this example has characteristic peaks expressed at 20 angles consistent with those of Example 1. An X-ray diffraction pattern is essentially shown in FIG. 1.

The bioactive glass material belongs to a Na₂O-CaO-SiO₂-P₂O₅ system, and includes, in percentage by weight: 40% of SiO₂, 5% of P₂O₅, 27% of CaO, and 28% of Na₂O.

### Example 3

(1) Sintering of calcium carbonate: A quantity of analytically pure calcium carbonate was weighed, placed in a muffle furnace for calcination at 1,000 °C, and then cooled to room temperature to achieve a conversion rate of 100% to obtain sintered calcium oxide. A calculation formula for the conversion rate is as follows: Y = △m/44%m, wherein △m refers to a mass difference of the calcium carbonate before and after the calcination; and m refers to a mass of the calcium carbonate.
(2) A metal salt base solution at a volume proportion of 25% (based on a reactor volume of 5 L) was added into a reaction tank, wherein the metal salt base solution was sodium sulfate, and based on generated sodium oxide as sodium introduced, the sodium oxide accounted for 1.3% of a total mass of a bioactive glass material; and at a reaction temperature of 70 °C, simultaneous acid-base titration was performed, wherein a pH was maintained at 4.5 during the simultaneous acid-base titration, and a reaction time was 1 h. A base used was sodium silicate with a modulus of 3.2 and a concentration of 1.8 N, and based on silica generated by the alkali metal silicate accounting for 55% of the total mass of the bioactive glass material, a corresponding mass of the alkali metal silicate was added. An acid used was sulfuric acid with a concentration of 6 N.
(3) After completion of the reaction, the sintered calcium oxide obtained in step (1) was added, with an addition amount of the sintered calcium oxide accounting for 22% of the total mass of the bioactive glass material, and then stirred evenly at a rotation speed of 600 rpm for 20 min; subsequently, diluted phosphoric acid was added for a reaction time of 0.4 h, wherein a concentration of the diluted phosphoric acid was 35%, and based on generated phosphorus pentoxide accounting for 3% of the total mass of the bioactive glass material, a corresponding mass of the diluted phosphoric acid was added; after completion of the reaction, stirring was performed continuously at a rotation speed of 600 rpm for 30 min, and subsequently, sodium hydroxide was added, wherein based on generated sodium oxide as sodium introduced by the sodium hydroxide, the sodium oxide accounted for 1% of the total mass of the bioactive glass material; and stirring was performed continuously at a rotation speed of 600 rpm for 30 min, followed by discharging.
(4) A material solution discharged in the step (3) was subjected to spray drying at an air inlet temperature of 300 °C and an air outlet temperature of 120 °C, and then crushed to a required particle size to obtain the bioactive glass material.

After analysis and identification by X-ray diffraction (XRD) using a Bruker D8A A25 X-ray diffractometer with Cu-Kα radiation, the bioactive glass material prepared in this example has characteristic peaks expressed at 20 angles consistent with those of Example 1. An X-ray diffraction pattern is essentially shown in FIG. 1.

The bioactive glass material belongs to a Na₂O-CaO-SiO₂-P₂O₅ system, and includes, in percentage by weight: 55% of SiO₂, 3% of P₂O₅, 22% of CaO, and 20% of Na₂O.

### Example 4

(1) Sintering of calcium carbonate: A quantity of analytically pure calcium carbonate was weighed, placed in a muffle furnace for calcination at 1,000 °C, and then cooled to room temperature to achieve a conversion rate of 100% to obtain sintered calcium oxide. A calculation formula for the conversion rate is as follows: Y = △m/44%m, wherein △m refers to a mass difference of the calcium carbonate before and after the calcination; and m refers to a mass of the calcium carbonate.
(2) A metal salt base solution at a volume proportion of 30% (based on a reactor volume of 5 L) was added into a reaction tank, wherein the metal salt base solution was sodium sulfate, and based on generated sodium oxide as sodium introduced, the sodium oxide accounted for 4% of a total mass of a bioactive glass material; and at a reaction temperature of 60 °C, simultaneous acid-base titration was performed, wherein a pH was maintained at 5.5 during the simultaneous acid-base titration, and a reaction time was 1 h. A base used was sodium silicate with a modulus of 3.1 and a concentration of 2.2 N, and based on silica generated by the alkali metal silicate accounting for 48% of the total mass of the bioactive glass material, a corresponding mass of the alkali metal silicate was added. An acid used was sulfuric acid with a concentration of 10 N.
(3) After completion of the reaction, the sintered calcium oxide obtained in step (1) was added, with an addition amount of the sintered calcium oxide accounting for 23% of the total mass of the bioactive glass material, and then stirred evenly at a rotation speed of 600 rpm for 30 min; subsequently, diluted phosphoric acid was added for a reaction time of 0.3 h, wherein a concentration of the diluted phosphoric acid was 40%, and based on generated phosphorus pentoxide accounting for 6% of the total mass of the bioactive glass material, a corresponding mass of the diluted phosphoric acid was added; after completion of the reaction, stirring was performed continuously at a rotation speed of 600 rpm for 30 min, and subsequently, sodium hydroxide was added, wherein based on generated sodium oxide as sodium introduced by the sodium hydroxide, the sodium oxide accounted for 3% of the total mass of the bioactive glass material; and stirring was performed continuously at a rotation speed of 600 rpm for 30 min, followed by discharging.
(4) A material solution discharged in the step (3) was subjected to spray drying at an air inlet temperature of 300 °C and an air outlet temperature of 120 °C, and then crushed to a required particle size to obtain the bioactive glass material.

After analysis and identification by X-ray diffraction (XRD) using a Bruker D8A A25 X-ray diffractometer with Cu-Kα radiation, the bioactive glass material prepared in this example has characteristic peaks expressed at 20 angles consistent with those of Example 1. An X-ray diffraction pattern is essentially shown in FIG. 1.
The bioactive glass material belongs to a Na₂O-CaO-SiO₂-P₂O₅ system, and includes, in percentage by weight: 48% of SiO₂, 6% of P₂O₅, 23% of CaO, and 23% of Na₂O. Detected physicochemical indicators of the bioactive glass materials prepared and obtained in Examples 1 to 4 are shown in Table 1 below.

**Table 1**

| | Example 1 | Example 2 | Example 3 | Example 4 |
|---|---|---|---|---|
| Apparent density (g/ml) | 0.67 | 0.6 | 0.55 | 0.5 |
| Copper consumption (mg) | 3.5 | 3 | 2.5 | 2 |
| Ganz whiteness | 99.3 | 98.9 | 99.1 | 99.2 |
| Moisture (%) | 0.5 | 0.3 | 0.6 | 0.4 |
| D50 particle size (µm) | 3.8 | 3.5 | 3.7 | 3.9 |
| BET (m²/g) | 10 | 13 | 16 | 20 |
| Pore volume (cm³/g) | 0.1 | 0.11 | 0.12 | 0.13 |
| Pore size (nm) | 35 | 36 | 37 | 38 |
| Oil absorption value (g/100 g) | 70 | 75 | 80 | 85 |
| pH | 8.5 | 9 | 9.5 | 10 |

### Example 5

(1) Sintering of calcium carbonate: A quantity of analytically pure calcium carbonate was weighed, placed in a muffle furnace for calcination at 1,000 °C, and then cooled to room temperature to achieve a conversion rate of 100% to obtain sintered calcium oxide. A calculation formula for the conversion rate is as follows: Y = △m/44%m, wherein △m refers to a mass difference of the calcium carbonate before and after the calcination; and m refers to a mass of the calcium carbonate.
(2) A metal salt base solution at a volume proportion of 20% (based on a reactor volume of 5 L) was added into a reaction tank, wherein the metal salt base solution was sodium sulfate, and based on generated sodium oxide as sodium introduced, the sodium oxide accounted for 5.7% of a total mass of a bioactive glass material; and at a reaction temperature of 80 °C, simultaneous acid-base titration was performed, wherein a pH was maintained at 8 during the process, and a reaction time was 1.2 h. A base used was sodium silicate with a modulus of 3.5 and a concentration of 2.2 N, and based on silica generated by the alkali metal silicate accounting for 45% of the total mass of the bioactive glass material, a corresponding mass of the alkali metal silicate was added. An acid used was phosphoric acid with a concentration of 6 N, and generated phosphorus pentoxide accounted for 6% of the total mass of the bioactive glass material.
   After completion of the reaction, the sintered calcium oxide obtained in step (1) was added, with an addition amount of the sintered calcium oxide accounting for 24% of the total mass of the bioactive glass material, and then stirred evenly at a rotation speed of 600 rpm for 20 min; subsequently, sodium hydroxide was added, wherein based on generated sodium oxide as sodium introduced by the sodium hydroxide, the sodium oxide accounted for 6% of the total mass of the bioactive glass material; and stirring was performed continuously at a rotation speed of 600 rpm for 30 min, followed by discharging.
(3) A material solution discharged was subjected to spray drying at an air inlet temperature of 300 °C and an air outlet temperature of 120 °C, and then crushed to a required particle size to obtain the bioactive glass material.

After analysis and identification by X-ray diffraction (XRD) using a Bruker D8A A25 X-ray diffractometer with Cu-Kα radiation, the bioactive glass material prepared in this example has characteristic peaks expressed at the following 2θ angles: 2θ=20.7±0.2°, 2θ=22.0±0.2°, 2θ=23.6±0.2°, 2θ=23.8±0.2°, 2θ=26.8±0.2°, 2θ=33.6±0.2°, 2θ=34.2±0.2°, 2θ=48.6±0.2°. An X-ray diffraction pattern of the bioactive glass material prepared in this example is shown in FIG. 4.

The bioactive glass material belongs to a Na₂O-CaO-SiO₂-P₂O₅ system, and includes, in percentage by weight: 45% of SiO₂, 6% of P₂O₅, 24% of CaO, and 25% of Na₂O.

### Example 6

(1) Formulation of a reaction material solution A: Calcium carbonate was calcined to obtain calcium oxide (referring to Example 1); the obtained calcium oxide was formulated into a calcium hydroxide slurry with a concentration of 0.8 mol/L, wherein based on the generated calcium oxide as calcium introduced, the calcium oxide accounted for 6% of a total mass of a bioactive glass material; heating was performed up to 85°C, and a diluted phosphoric acid solution was added dropwise into the calcium hydroxide slurry, wherein based on generated phosphorus pentoxide accounting for 4% of the total mass of the bioactive glass material, a corresponding mass of the diluted phosphoric acid was added, a concentration of the diluted phosphoric acid solution was 38%, a reaction pH was 7-9, and a reaction time was 2 h; and after completion of the reaction, aging and standing were performed at normal temperature for 2 h to obtain the reaction material solution for later use.
(2) Formulation of a reaction material solution B: A silica slurry at a volume proportion of 10% (based on a reactor volume of 5 L) was added into a reaction tank, wherein based on generated silica as silicon introduced, the silica accounted for 60% of the total mass of the bioactive glass material; subsequently, the calcined calcium oxide was added, with the calcined calcium oxide accounting for 12% of the total mass of the bioactive glass material, and stirred at a rotation speed of 600 rpm for 30 min; then, sodium hydroxide was added, wherein based on generated sodium oxide as sodium introduced by the sodium hydroxide, the sodium oxide accounted for 18% of the total mass of the bioactive glass material; and stirring was performed evenly at a rotation speed of 600 rpm for a stirring time of 4 h to obtain the reaction material solution for later use.
(3) The reaction materials solution A and the reaction materials solution B were evenly mixed and stirred, dried in an oven at 110 °C until a moisture content was less than 3%, and then crushed to obtain the bioactive glass material.

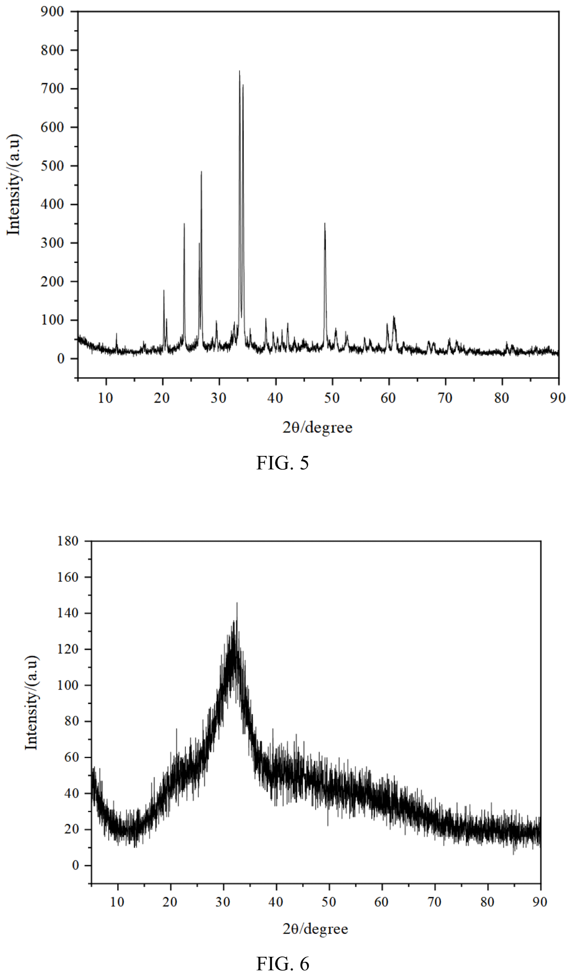

After analysis and identification by X-ray diffraction (XRD) using a Bruker D8A A25 X-ray diffractometer with Cu-Kα radiation, the bioactive glass material prepared in this example has characteristic peaks expressed at the following 2θ angles: 2θ=20.2±0.2°, 2θ=23.8±0.2°, 2θ=26.5±0.2°, 2θ=26.8±0.2°, 2θ=33.6±0.2°, 2θ=34.2±0.2°, 2θ=48.6±0.2°. An X-ray diffraction pattern of the bioactive glass material prepared in this example is shown in FIG. 5.

The bioactive glass material belongs to a Na₂O-CaO-SiO₂-P₂O₅ system, and includes, in percentage by weight: 60% of SiO₂, 4% of P₂O₅, 18% of CaO, and 18% of Na₂O.

### Comparative Example 1

In this comparative example, existing bioglass Novamin4516 is used, which includes, in percentage by weight: 24.5 wt% of Na₂O, 24.5 wt% of CaO, 6.0 wt% of P₂O₅, and 45 wt% of SiO₂.

The bioglass Novamin4516 is analyzed and identified by X-ray diffraction (XRD) using a Bruker D8A A25 X-ray diffractometer with Cu-Kα radiation. An X-ray diffraction pattern of the bioglass Novamin4516 is shown in FIG. 6. It can be seen from FIG. 6 that the bioglass Novamin4516 does not have characteristic peaks in the X-ray diffraction pattern and is of an amorphous structure.

### Comparative Example 2

A bioactive glass material used in this comparative example is prepared in the form of powder according to a method described in Example 2 of a patent application with patent publication No. CN111017934A.

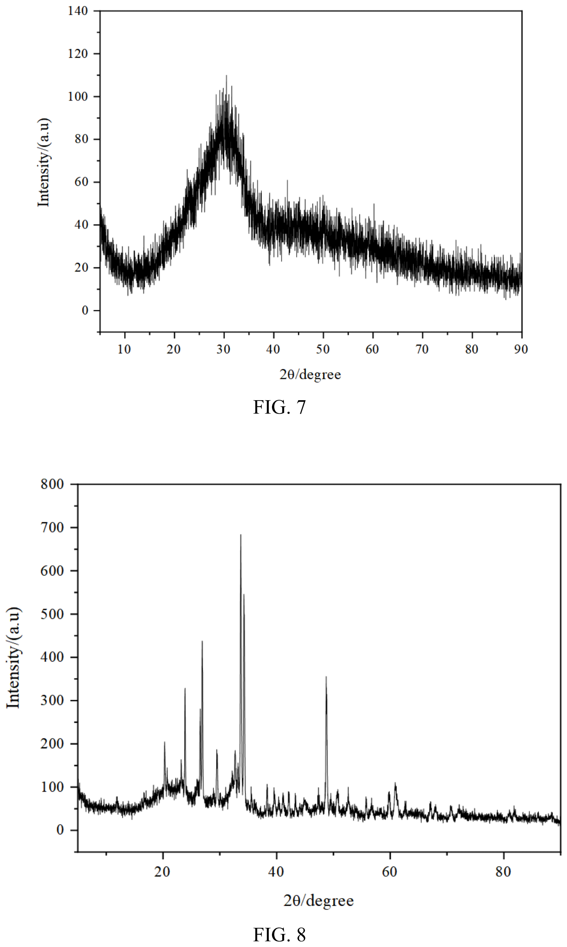

X-ray diffraction (XRD) analysis and identification are performed using a Bruker D8A A25 X-ray diffractometer with Cu-Kα radiation. An X-ray diffraction pattern of the bioactive glass material of Comparative Example 2 is shown in FIG. 7. It can be seen from FIG. 7 that the bioactive glass material prepared in this comparative example does not have characteristic peaks in the X-ray diffraction pattern and is of an amorphous structure.

### Test Example

### (1) Artificial saliva mineralization experiment

An artificial saliva mineralization experiment was conducted on the bioactive glass prepared in Example 1, Comparative Example 1, and Comparative Example 2. Experimental steps: 0.60 g of a sample was weighed and added into a 100 mL beaker, commercially available artificial saliva was added to reach a scale of 100 mL, a resulting mixture was stored at a constant temperature of 37 °C, and the artificial saliva was replaced every 24 h. The experiment was conducted for 1 day, 3 days, and 7 days, respectively. Experimental samples were numbered as A, B, and C, and respectively subjected to energy spectrum analysis to detect changes in Ca/P molar content ratio. Meanwhile, an XRD test was conducted on reaction products.

If, as the artificial saliva mineralization experiment proceeds, the Ca/P molar content ratio gradually decreases with the increase of artificial saliva mineralization time, it is indicated that the phosphorus element in the saliva undergoes a combination reaction with the calcium element in the bioglass. If the Ca/P molar content ratio gradually approaches 1.67, it is inferred that hydroxyapatite may be generated.

**Table 2**

| Artificial saliva mineralization time | 1 d | 2 d | 3 d |
|---|---|---|---|
| Example 1 | 2.759 | 1.96 | 1.68 |
| Comparative Example 1 | 2.964 | 2.124 | 1.81 |
| Comparative Example 2 | 2.925 | 3.2 | 2.536 |

Artificial saliva mineralization experiment results are shown in Table 2 above. It can be seen from Table 2 that the Ca/P molar content ratio of the bioactive glass prepared in Example 1 of the present invention gradually approaches 1.67 with the increase of artificial saliva mineralization time, inferring that the hydroxyapatite may be generated. However, the Ca/P molar content ratio of the bioactive glass of Comparative Example 1 and Comparative Example 2 after artificial saliva mineralization still has a distance from 1.67.

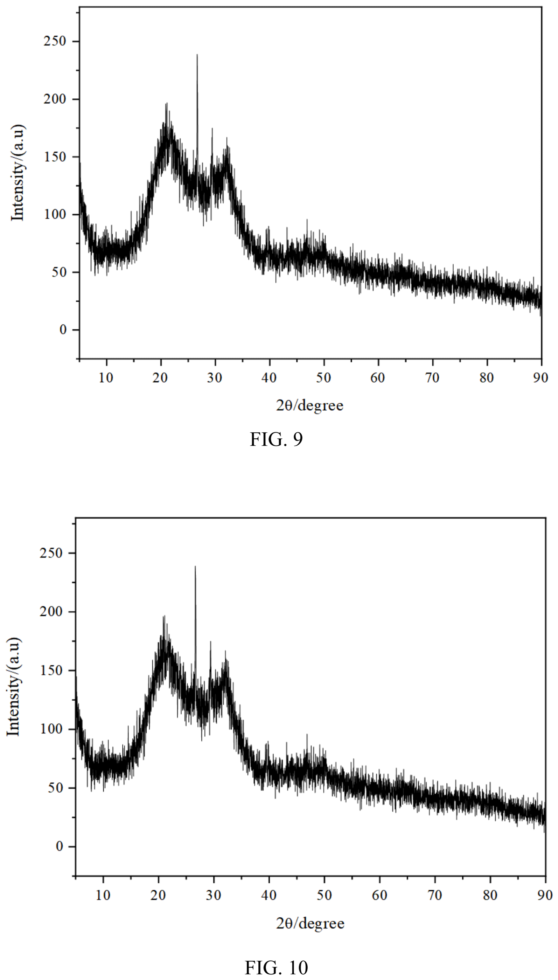

X-ray diffraction patterns of the products obtained after the artificial saliva mineralization of the bioactive glass materials in Example 1, Comparative Example 1, and Comparative Example 2 are shown in FIG. 8, FIG. 9, and FIG. 10, respectively. It can be seen from FIG. 8 that the product obtained after the artificial saliva mineralization of the bioactive glass material prepared in Example 1 has a characteristic peak at 2θ=34.2358° in the X-ray diffraction pattern, which corresponds to a characteristic diffraction peak of the hydroxyapatite, indicating that the bioactive glass material provided by the present invention generates the hydroxyapatite after the artificial saliva mineralization. It can be seen from FIG. 9 and FIG. 10 that the products obtained after the mineralization of the bioactive glass in Comparative Example 1 and Comparative Example 2 do not have characteristic peaks and are amorphous products without the generation of hydroxyapatite crystals.

### (2) Dentinal tubule mineralization and occlusion experiment using bioglass toothpaste

Bioglass toothpaste includes, in percentage by weight: 0.95% of carbomer 940, 21.25% of PEG-400, 0.2% of sodium saccharin, 0.2% of methylparaben, 55% of glycerol, 3% of ZI-165, 11% of core-shell silica, 2% of K12, 5% of a bioactive glass material, 0.5% of titanium dioxide, and 0.9% of an essence.

### 1. Test method:

1.1 Preparation of dentin samples: Freshly removed bovine incisors were cut and polished to prepare 12 dentin blocks with a size of approximately 5 mm × 5 mm × 2 mm, and the dentin blocks were etched with 40% orthophosphoric acid for 20 min, rinsed, then etched with 5% sodium hypochlorite for 5 min, and finally ultrasonically cleaned with deionized water for 20 min. The dentin blocks were observed under a polarization microscope to ensure sufficient exposure of dentinal tubules.

1.2 Grouping: The 12 dentin samples were randomly divided into a blank control group, an experimental group 1, an experimental group 2, and an experimental group 3, with 3 blocks in each group.

1.3 Preparation of a toothpaste slurry: Certain amounts of toothpaste and artificial saliva were weighed according to a ratio of 1:1.6 (toothpaste:saliva), wherein the toothpaste was weighed directly on a toothbrush head, and the toothbrush head used was a basic ORALB brush head.

1.4 Partitioning: The dentin samples were pasted at half areas (approximately 5 mm × 2.5 mm) using an adhesive tape to serve as untreated areas, while the other half areas (approximately 5 mm × 2.5 mm) were served as experimental treatment areas.

1.5 Cyclic dentin treatment: The blank control group was treated with deionized water; the experimental group 1 was treated with bioglass toothpaste prepared from the bioactive glass material prepared in Example 1; the experimental group 2 was treated with bioglass toothpaste prepared from the bioactive glass material of Comparative Example 2; and the experimental group 3 was treated with bioglass toothpaste prepared from the bioactive glass material prepared in Comparative Example 1. The dentin samples were brushed with an electric toothbrush for 3 min, and then cleaned thoroughly with deionized water, followed by soaking in artificial saliva and heat preservation at 37 °C. The dentin samples were repeatedly brushed for 3 min, and then cleaned thoroughly with deionized water, followed by soaking in artificial saliva 2 times/d; and the dentin samples after brushing for 1 d were selected and observed using a scanning electron microscope.

1.6 Drying: The dentin samples after the cyclic treatment were dried in a constant-temperature chamber at 25 °C for 24 h.

1.7 Observation using a scanning electron microscope: The dried dentin was subjected to metal spraying, and then observed under the scanning electron microscope to obtain situations before and after occlusion.

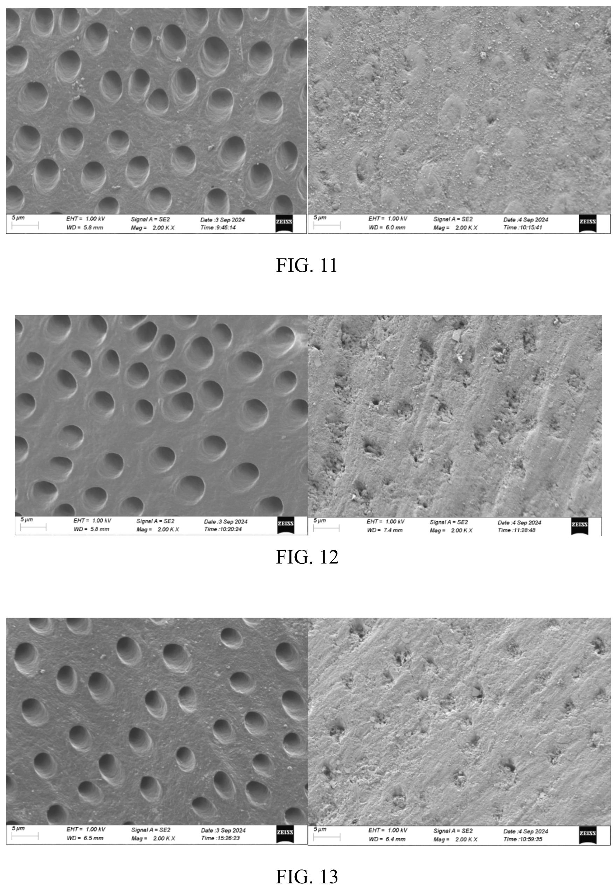

Experimental images of the dentinal tubule mineralization and occlusion experiment using the bioglass toothpaste prepared from the bioactive glass materials of Example 1, Comparative Example 1, and Comparative Example 2 are shown in FIG. 11, FIG. 12, and FIG. 13, respectively. It can be seen from FIG. 11, FIG. 12, and FIG. 13 that the bioglass toothpaste prepared from the bioactive glass material prepared in Example 1 has a significantly better mineralization and occlusion effect on the dentinal tubules than those of Comparative Example 1 and Comparative Example 2, and "bulges" are formed in Example 1. Great occlusion of the dentinal tubules is not achieved in Comparative Example 1 and Comparative Example 2, openings of some dentinal tubules are still exposed to the outside, and an occlusion effect of Comparative Example 1 is significantly inferior to that of Comparative Example 2.

### (3) Bioglass toothpaste and tooth surface reaction rate experiment

0.50 g of the bioactive glass materials of Example 1, Comparative Example 1, and Comparative Example 2 were respectively weighed and added into a 50 mL centrifuge tube, deionized water was added to reach a scale of 50 mL, and a resulting mixture was stored at a constant temperature of 37 °C; the mixture was centrifuged at different time points, and a supernatant was collected to detect the contents of silicon, calcium, phosphorus, and sodium elements therein; and the concentrations of silicon, calcium, phosphorus, and sodium elements in a bioglass aqueous solution with a concentration of 0.01% at different time points were analyzed using a plasma spectrometer. Results of Example 1, Comparative Example 1, and Comparative Example 2 are shown in Tables 3, 4, and 5, respectively.

**Table 3**

| Time | Silicon (ppm) | Calcium (ppm) | Phosphorus (ppm) | Sodium (ppm) |
|---|---|---|---|---|
| 1 h | 15.3 | 5.3 | 2.4 | 8.3 |
| 2 h | 18.1 | 7.6 | 2.0 | 10.1 |
| 4 h | 19.7 | 9.1 | 1.9 | 11.4 |
| 8 h | 20.5 | 10.9 | 1.5 | 14.8 |
| 12 h | 24.8 | 14.7 | 1.1 | 15.2 |
| 24 h | 25.7 | 15.5 | 0.6 | 16.8 |

**Table 4**

| Time | Silicon (ppm) | Calcium (ppm) | Phosphorus (ppm) | Sodium (ppm) |
|---|---|---|---|---|
| 1 h | 9.2 | 3.1 | 1.0 | 5.3 |
| 2 h | 11.5 | 4.4 | 1.4 | 6.1 |
| 4 h | 13.8 | 6.7 | 1.1 | 9.5 |
| 8 h | 15.9 | 8.9 | 0.9 | 11.7 |
| 12 h | 15.1 | 9.6 | 0.8 | 13.9 |
| 24 h | 16.3 | 10.2 | 0.7 | 14.8 |

**Table 5**

| Time | Silicon (ppm) | Calcium (ppm) | Phosphorus (ppm) |
|---|---|---|---|
| 1 h | 13.4 | 4.2 | 1.4 |
| 2 h | 14.3 | 6.8 | 1.8 |
| 4 h | 16.5 | 9.3 | 1.9 |
| 8 h | 17.8 | 13.9 | 1.0 |
| 12 h | 19.5 | 17.4 | 0.8 |
| 24 h | 22.9 | 20.7 | 0.6 |

It can be seen from above Tables 3, 4, and 5 that the bioglass toothpaste formed from the bioactive glass material prepared in the present invention, at 1 h, has a silicon element dissolution concentration of 15.3 ppm, a calcium element dissolution concentration of 5.3 ppm, a phosphorus element dissolution concentration of 2.4 ppm, and a sodium element dissolution concentration of 8.3 ppm. At 12 h, the silicon element dissolution concentration is 24.8 ppm, the calcium element dissolution concentration is 15.5 ppm, the phosphorus element dissolution concentration is 0.6 ppm, and the sodium element dissolution concentration is 16.8 ppm.

In Comparative Example 1, the bioactive glass material, at 1 h, has a silicon element dissolution concentration of 9.2 ppm, a calcium element dissolution concentration of 3.1 ppm, a phosphorus element dissolution concentration of 1.0 ppm, and a sodium element dissolution concentration of 5.3 ppm. The dissolution rate of the bioactive glass material of Comparative Example 1 at 1 h is much lower than that of the bioactive glass material of the present invention.

In Comparative Example 2, the bioactive glass material, at 1 h, has a silicon element dissolution concentration of 13.4 ppm, a calcium element dissolution concentration of 4.2 ppm, and a phosphorus element dissolution concentration of 1.4 ppm. The dissolution rate of the bioactive glass material of the present invention is also superior to that of the bioactive glass material of Comparative Example 2.

The above results show that compared with existing bioactive glass, the bioactive glass material provided by the present invention has superior dissolution rates for the silicon element, the calcium element, the phosphorus element, and the sodium element, and thus can generate a silica glass network in water or body fluid more quickly. The calcium element and the phosphorus element can also be released to a tooth surface more quickly to crystallize into mixed hydroxy-carbonate apatite on the surface. The mixed hydroxy-carbonate apatite is deposited on the tooth surface to achieve a mineralization effect to occlude the exposed dentinal tubules, which can react with the tooth surface more quickly to achieve remineralization of a dentin surface, thereby playing the role of alleviating or treating dentin hypersensitivity.

The above examples are preferred embodiments of the present invention, but the embodiments of the present invention are not limited to the above examples. Any other alterations, modifications, substitutions, combinations, and simplifications made without departing from the spiritual essence and principles of the present invention shall be regarded as equivalent replacement modes and are all included within the scope of protection of the present invention.

## Claims

1. A bioactive glass material, wherein the bioactive glass material belongs to a Na₂O-CaO-SiO₂-P₂O₅ system, and comprises, in percentage by weight: 40-60% of SiO₂, 1-10% of P₂O₅, 18-30% of CaO, and 10-30% of Na₂O; and an X-ray diffraction pattern of the bioactive glass material comprises diffraction peaks at the following 20 angle values: 20.2±0.2°, 23.8±0.2°, 26.5±0.2°, 26.8±0.2°, 33.6±0.2°, 34.2±0.2°, and 48.6±0.2°.

2. The bioactive glass material according to claim 1, wherein the X-ray diffraction pattern of the bioactive glass material comprises diffraction peaks at the following 20 angle values: 19.1±0.2°, 20.2±0.2°, 20.7±0.2°, 22.0±0.2°, 23.6±0.2°, 23.8±0.2°, 26.5±0.2°, 26.8±0.2°, 26.9±0.2°, 31.9±0.2°, 32.1±0.2°, 33.6±0.2°, 34.2±0.2°, and 48.6±0.2°.

3. The bioactive glass material according to claim 1, wherein the bioactive glass material has an X-ray diffraction pattern consistent with FIG. 1, FIG. 4, or FIG. 5.

4. The bioactive glass material according to claim 1, wherein a pH of the bioactive glass material is 8.5-10.

5. The bioactive glass material according to claim 1, comprising at least one of the following:
(a) an apparent density of the bioactive glass material being 0.5-1.2 g/mL;
(b) the bioactive glass material comprising 0.3-2.0% of moisture;
(c) a D50 particle size of the bioactive glass material being 3.5-4.0 µm;
(d) a Brunauer-Emmett-Teller (BET) specific surface area of the bioactive glass material being 10-20 m²/g;
(e) a pore volume of the bioactive glass material being 0.01-0.2 cm³/g;
(f) a pore size of the bioactive glass material being 35-40 nm;
(g) an oil absorption value of the bioactive glass material being 20-100 g/100 g;
(h) a copper consumption value of the bioactive glass material being 2-4 mg; and
(i) a Ganz whiteness of the bioactive glass material being > 98.

6. The bioactive glass material according to claim 1, wherein the bioactive glass material is prepared and obtained by a first preparation method, a second preparation method, or a third preparation method;
the first preparation method comprises the following steps:
(1) subjecting an alkali metal silicate and an inorganic acid to simultaneous acid-base titration, and during the simultaneous titration, maintaining a solution pH at 2-6 and a reaction temperature at 40-95 °C; or
subjecting an alkali metal silicate and an inorganic acid to simultaneous acid-base titration in a metal salt base solution, and during the simultaneous titration, maintaining a solution pH at 2-6 and a reaction temperature at 40-95 °C; and
(2) after completion of the reaction in step (1), adding calcium oxide, and stirring; adding phosphoric acid for a reaction, and stirring; and then adding a sodium-containing alkali solution, and stirring to prepare and obtain the bioactive glass material;
the second preparation method comprises the following steps:
(1) subjecting an alkali metal silicate and phosphoric acid to simultaneous acid-base titration, and during the simultaneous titration, maintaining a solution pH at 3-10 and a reaction temperature at 40-95 °C; or
subjecting an alkali metal silicate and phosphoric acid to simultaneous acid-base titration in a metal salt base solution, and during the simultaneous titration, maintaining a solution pH at 3-10 and a reaction temperature at 40-95 °C; and
(2) after completion of the reaction in step (1), adding calcium oxide, and stirring; and then adding a sodium-containing alkali solution, and stirring to prepare and obtain the bioactive glass material; and
the third preparation method comprises the following steps:
S1. at 40-95 °C, mixing a calcium hydroxide slurry and phosphoric acid for a reaction, and maintaining a pH at 7-9 during the reaction; and after completion of the reaction, performing aging treatment to obtain a reaction material solution A;
S2. mixing a silica slurry and calcium oxide, and stirring; and then adding a sodium-containing alkali solution, and stirring to obtain a reaction material solution B; and
S3. mixing the reaction material solution A and the reaction material solution B, and drying to obtain the bioactive glass material.

7. Use of the bioactive glass material according to claim 1 in occlusion of a dentinal tubule or in preparation of an oral care composition.

8. An oral care composition, comprising, in percentage by weight: 0.1-20% of the bioactive glass material according to claim 1 and an orally acceptable auxiliary material.

9. The oral care composition according to claim 8, wherein the oral care composition is toothpaste.

10. The oral care composition according to claim 9, wherein the toothpaste comprises, in parts by weight: 1-10 parts of the bioactive glass material, 20.5-26.5 parts of a thickener, 45-60 parts of a humectant, 11-20 parts of an abrasive, 1-5 parts of a foaming agent, 0-3 parts of a colorant, 0-3 parts of a flavoring agent, 0-0.5 part of a sweetener, and 0-0.5 part of an antimicrobial agent.

11. The oral care composition according to claim 9, wherein the toothpaste comprises, in percentage by weight: 0.95% of carbomer 940, 21.25% of PEG-400, 0.2% of sodium saccharin, 0.2% of methylparaben, 55% of glycerol, 3% of ZI-165, 11% of core-shell silica, 2% of K12, 5% of the bioactive glass material, 0.5% of titanium dioxide, and 0.9% of an essence.

12. The oral care composition according to claim 8, wherein an X-ray diffraction pattern of the bioactive glass material comprises diffraction peaks at the following 20 angle values: 19.1±0.2°, 20.2±0.2°, 20.7±0.2°, 22.0±0.2°, 23.6±0.2°, 23.8±0.2°, 26.5±0.2°, 26.8±0.2°, 26.9±0.2°, 31.9±0.2°, 32.1±0.2°, 33.6±0.2°, 34.2±0.2°, and 48.6±0.2°.

13. The oral care composition according to claim 12, wherein the oral care composition is toothpaste.

14. The oral care composition according to claim 13, wherein the toothpaste comprises, in parts by weight: 1-10 parts of the bioactive glass material, 20.5-26.5 parts of a thickener, 45-60 parts of a humectant, 11-20 parts of an abrasive, 1-5 parts of a foaming agent, 0-3 parts of a colorant, 0-3 parts of a flavoring agent, 0-0.5 part of a sweetener, and 0-0.5 part of an antimicrobial agent.

15. The oral care composition according to claim 13, wherein the toothpaste comprises, in percentage by weight: 0.95% of carbomer 940, 21.25% of PEG-400, 0.2% of sodium saccharin, 0.2% of methylparaben, 55% of glycerol, 3% of ZI-165, 11% of core-shell silica, 2% of K12, 5% of the bioactive glass material, 0.5% of titanium dioxide, and 0.9% of an essence.
